Europäisches Patentamt

(19) European Patent Office

Office européen des brevets

(11) Publication number: **0 243 038 B1**

(12) **EUROPEAN PATENT SPECIFICATION**

(45) Date of publication of the patent specification:
22.11.90

(21) Application number: 87303070.4

(22) Date of filing: 09.04.87

(51) Int. Cl.⁵: **C07C 39/32,** C07C 37/62,
B01J 31/02

(54) **Preparation of trichlorophenol.**

(30) Priority: 23.04.86 GB 8609904

(43) Date of publication of application:
28.10.87 Bulletin 87/44

(45) Publication of the grant of the patent:
22.11.90 Bulletin 90/47

(84) Designated Contracting States:
AT BE CH DE ES FR GB GR IT LI LU NL SE

(56) References cited:
DE-A- 2 632 565
GB-A- 2 135 310
US-A- 4 250 341

BEILSTEINS HANDBUCH DER ORGANISCHEN CHEMIE,
vol. 6, 4th edition, 1923, pages 190-191, Springer
Publicationer, Berlin;
CHEMICAL ABSTRACTS, vol. 63, no. 6, 13th
September 1965, column 6897, Columbus, Ohio, US;
V.A. NEKRASOVA et al.: "Chlorination of phenol", & ZH.
PRIKL. KHIM. 1965, 38(6), 1407-1409
PATENT ABSTRACTS OF JAPAN, vol. 1,
no. 64 (C-77), 22nd June 1977, page 973 C 77; & JP - A
- 52 27734 (SUMITOMO KAGAKU KOGYO
K.K.) 03-02-1977

(73) Proprietor: SCHERING AGROCHEMICALS LIMITED,
Hauxton Cambridge CB2 5HU(GB)

(72) Inventor: Cole, Graham Martin, 227 Cherry Hinton Road,
Cambridge England(GB)
Inventor: Jackson, Timothy Harry, Harshel Court
Hartington Grove, Cambridge England(GB)
Inventor: Taylor, Robert William, Norris Close 21 King
Street, Rampton, Cambridge England(GB)
Inventor: Foreman, Peter James, 3 Hauxton Road,
Trumpington, Cambridge England(GB)

(74) Representative: Waldman, Ralph David et al, Schering
Agrochemicals Limited Industrial Property Department
Chesterford Park Research Station, Saffron Walden
Essex CB10 1XL(GB)

## Description

This invention relates to an improved process for preparing 2,4,6-trichlorophenol and salts thereof.

2,4,6-Trichlorophenol is used as an intermediate in the manufacture, inter alia, of the fungicide known by the common name, prochloraz, which is N-propyl-N-[2-(2,4,6-trichlorophenoxy)ethyl]imidazole-1-carboxamide. One of the steps in the manufacture of prochloraz involves converting the phenol to its sodium salt. It is essential that the 2,4,6-trichlorophenol is used in a highly pure form, since trace impurities can be converted into solid and tarry by-products both in the preparation of the sodium phenate and in later reaction stages. In GB 2 135 310, there is diclosed as prior art, that 2,4,6-trichlorophenol can be produced by chlorination of of phenol to until it is dichlorophenol and at this stage a catalyst such as aluminium or ferric chloride to assist in accelerating the chlorination. When the reaction is complete the mixture is air-blown to remove hydrogen chloride and then distilled to purify it from unwanted by-products. This distillation is expensive because it is a highly capital and energy intensive operation. In addition it leads to a ca. 25% loss of product. In order to reduce the amount of residue, it is proposed in this patent specification, that the catalyst should be omitted. However a distillation stage is still required and apart from the already mentioned disadvantages, the high temperature required for distillation also leads to the formation of polychlorodibenzo-p-dioxins.

We have now found that high purity 2,4,6-trichloro-phenol can be prepared by a process which comprises the slow controlled chlorination of phenol over a period of at least 10 hours, at a temperature of 40 to 80°C, and in the presence of an organophosphorus chlorination catalyst, to give 2,4,6-trichlorophenol of a purity of at least 95%.

In a further embodiment of the invention this 2,4,6-trichlorophenol can then be used to obtain a high purity metal, and especially sodium, 2,4,6-trichloro-phenolate by treating it with a metal hydroxide in the presence of a reducing agent.

During a slow controlled chlorination the conditions of temperature and rate of addition of chlorine are such that overchlorination is avoided so that large quantitites of higher chlorinated phenols are not formed. The chlorination reaction is preferably carried out at a temperature of 60 to 75°C. Suitable organophosphorus catalysts are described in our British Patent 2 036 720 and especially preferred is triphenylphosphine oxide. In that patent, the catalysts are used to aid the chlorination of a cyclohexanol. There is nothing to suggest that the use of these catalysts for preparing 2,4,6-trichlorophenol from phenol would give the high quality product obtained according to the invention. These catalysts also have the advantage that they are easily removed during solvent washing stages either in the isolation of the 2,4,6-trichlorophenol itself or in subsequent processing in which the 2,4,6-trichlorophenol is used. In particular their presence in the subsequent treatment with the alkali metal hydroxide causes no problem, whereas it would be very difficult to separate the aluminium or ferric chloride catalysts, previously mentioned, and if they are present during treatment with the alkali metal hydroxide flocculant hydroxide precipitates are formed which are obviously highly undesirable and also difficult to remove.

In conventional chlorination of phenol it is usually necessary to use stoichiometrically excessive amounts of chlorine to form the trichlorophenol. This results in the formation of unwanted by-products, such as polychlorinated phenoxyphenols and 2,3,4,6-tetrachlorophenol, by overchlorination. The use of the particular chlorination catalyst and addition of chlorine at a relatively slow rate leads to the formation of high purity 2,4,6-trichlorophenol. The catalyst is generally present in an amount of 0.1 to 1% by weight of phenol.

Desirably, the reaction waste gases are passed through molten phenol in a secondary reactor eg. at 40 to 70°C, preferably 45 to 50°C, to ensure efficient use of chlorine and to remove trace amounts of chlorophenols. This material forms the next batch for chlorination. The end of the reaction can be generally determined by g.l.c. assay. The 2,4,6-trichlorophenol is generally converted immediately into its salt (usually the sodium salt) to prevent the formation of unwanted bi-products during storage as a melt.

Suitable reducing agents for this stage of the reaction include sodium sulphite and preferably sodium dithionite. These are usually present in an amount of 0.2 to 5% by weight of the trichlorophenol. Preferably molten trichlorophenol is added to dilute caustic soda containing the reducing agent. The addition is preferably carried out under an inert atmosphere. The temperature range is 20 to 75°C, preferably 30 to 55°C. Generally the redox potential during the addition is kept at less than −760mV but it is then allowed to rise to greater than −300mV by air sparging. The final required pH of the product is desirably between 10.0 and 10.5.

The invention is illustrated in the following example.

### Example

A primary chlorinator (hereinafter referred to as V1), containing phenol (93 kg) and triphenylphosphine oxide (0.18 kg), and a secondary chlorinator (hereinafter referred to as V2), containing phenol (93 kg), were nitrogen purged for one hour. The temperature of V1 contents were adjusted to about 60°C and that of V2 contents to about 50°C.

Chlorine was charged to V1 at a rate of 8.1 kg hour$^{-1}$. The waste gases from V1 were passed through the phenol in V2 . The temperature of V1 was kept ca. 60°C and that of V2 at ca. 50°C. After chlorine had been charged for 15 hours the temperature of V1 was allowed to rise to 72–75°C during the following 10 hours.

After 23 hours the V1 contents were sampled for gas chromatography assay. Chlorination was continued until further sampling indicated that the end of the reaction had been reached, after a total of 25.5 hours chlorination. The V1 contents were nitrogen sparged for 0.5 hour.

Water (153 litres) and 10M caustic soda (125 kg) were charged to a third reactor (V3.) V3 contents were nitrogen purged for 30 minutes and warmed to 45°C after which sodium dithionite (3.1 kg) was added.

2,4,6-Trichlorophenol was charged from V1 to V3 by gravity during about 15 minutes. Nitrogen purging was maintained. Cooling was applied to the jacket of V3 as soon as the 2,4,6-trichlorophenol addition had begun. The temperature of V3 contents rose from 46 to 51°C during the addition. The pH fell from greater than 14 to 9.0 and the redox potential rose from less than −700mV to −400mV just at the end of the addition. The pH was adjusted to 10.0 with 10M caustic soda (13.2 kg).

Nitrogen sparging was stopped and the contents of V3 air-sparged for 10 minutes to raise the redox potential to −280mV. V3 contents were cooled during this operation. Agitation of V3 contents was continued until the redox potential had stabilised. The final pH was 10.5 and so no further adjustment with caustic soda or hydrochloric acid was necessary.

The contents of V3 were transferred to a fourth reactor, V4. Toluene (369 litres) was charged to V4 and the mixture stirred for 30 minutes. V4 contents were allowed to settle for 30 minutes. The lower aqueous layer of high purity sodium 2,4,6-trichlorophenolate (493.1 kg) was transferred to drums. The product was a clear light tan to red-brown coloured solution free from tars and solids.

The product was subsequently used as a starting material to produce high quality prochloraz.

## Claims

1. A process for the preparation of high purity 2,4,6-trichlorophenol, which comprises the slow controlled chlorination of phenol over a period of at least 10 hours, at a temperature of 40 to 80°C, and in the presence of an organophosphorus chlorination catalyst, to give 2,4,6-trichlorophenol of a purity of at least 95%.

2. A process according to claim 1, in which the chlorination is carried out in the presence of triphenylphosphine oxide.

3. A process according to claim 1 or 2, which comprises the further step of subsequently treating the 2,4,6-trichlorophenol with a metal hydroxide in the presence of a reducing agent to give high purity metal 2,4,6-trichlorophenolate.

4. A process according to claim 3, in which the metal trichlorophenolate is sodium trichlorophenolate.

5. A process according to claim 4, in which the reducing agent is sodium dithionite.

## Patentansprüche

1. Verfahren zur Herstellung von hochreinem 2,4,6-Trichlorphenol, dadurch gekennzeichnet, daß die Chlorierung des Phenols kontrolliert langsam über einen Zeitraum von mindestens 10 Stunden bei einer Temperatur von 40–80°C in Gegenwart eines phosphororganischen Chlorierungskatalysators ausgeführt wird, wobei man ein 2,4,6-Trichlorphenol mit einer Reinheit von mindestens 95% erhält.

2. Verfahren nach Anspruch 1, dadurch gekennzeichnet, daß die Chlorierung in Gegenwart von Triphenylphosphinoxid durchgeführt wird.

3. Verfahren nach Anspruch 1 oder 2, dadurch gekennzeichnet, daß in einem weiteren Verfahrensschritt das 2,4,6-Trichlorphenol mit einem Metallhydroxid in Gegenwart eines Reduktionsmittels behandelt wird, wobei hochreines 2,4,6-Trichlor-metallphenolat erhalten wird.

4. Verfahren nach Anspruch 3, dadurch gekennzeichnet, daß das Trichlor-metallphenolat das Trichlor-natriumphenolat ist.

5. Verfahren nach Anspruch 4, dadurch gekennzeichnet, daß das Reduktionsmittel Natriumdithionit ist.

## Revendications

1. Procédé de préparation de 2,4,6-trichlorophénol de haute pureté qui comprend la chloration contrôlée lente du phénol en une durée d'au moins 10 heures à une température de 40 à 80°C et en présence d'un catalyseur de chloration organophosphoré pour former du 2,4,6-trichlorophénol d'une pureté d'au moins 95%.

2. Procédé suivant la revendication 1, dans lequel la chloration est exécutée en présence d'oxyde de triphénylphosphine.

3. Procédé suivant la revendication 1 ou 2, qui comprend le stade supplémentaire de traiter ensuite le 2,4,6-trichlorophénol avec un hydroxyde de métal en présence d'un agent réducteur pour obtenir du 2,4,6-trichlorophénate de métal de haute pureté.

4. Procédé suivant la revendication 3, dans lequel le trichlorophénate de métal est le trichlorophénate de sodium.

5. Procédé suivant la revendication 4, dans lequel l'agent réducteur est le dithionite de sodium.